# EUROPEAN PATENT APPLICATION

(11) **EP 3 360 549 A1**
(43) Date of publication of application: **15.08.2018**
(21) Application number: 17156089.9
(22) Date of filing: 14.02.2017
(51) Int. Cl.: A61K 31/34, A61K 31/7042, A61P 25/28

(54) **SMALL MOLECULE THERAPEUTICS FOR TREATING METACHROMATIC LEUKODYSTROPHY**

(71) Applicant: Rheinische Friedrich-Wilhelms-Universität Bonn, 53113 Bonn (DE)
(72) Inventor: Gieselmann, Volkmar, 53347 Alfter (DE); König, Gabriele Maria, 53127 Bonn (DE); Eckhardt, Matthias, 53127 Bonn (DE); Hufendiek, Peter, 81375 München (DE); Zech, Isabell, 53113 Bonn (DE)
(74) Representative: Kuttenkeuler, David

(57) **Abstract**

The present invention pertains to a novel treatment of pathologies caused by an increased synthesis or accumulation of sulfolipids such as sulfatide. The invention provides compounds isolated from *Epicoccum nigrum* strains 749 and 800, which were identified as inhibitors of Sulfotransferase, an enzyme catalyzing the generation of sulfatide. Sulfatides accumulate in the central nervous system (CNS) and lead to a degeneration of white matter. The present invention therefore preferably provides new therapeutic options for treating such diseases by administration of *Epicoccum nigrum* derived compounds. Further provided are pharmaceutical compositions comprising these compounds.

## Description

### FIELD OF THE INVENTION

The present invention pertains to a novel treatment of pathologies caused by an increased synthesis or accumulation of sulfolipids such as sulfatide. The invention provides compounds isolated from *Epicoccum nigrum* strains 749 and 800, which were identified as inhibitors of galactosyl ceramide sulfotransferase (EC 2.8.2.11), an enzyme catalyzing the generation of sulfatide. Sulfatides accumulate in the central nervous system (CNS) and peripheral nervous system (PNS) and lead to a degeneration of white matter. The present invention therefore preferably provides new therapeutic options for treating such diseases by administration of *Epicoccum nigrum* derived compounds. Further provided are pharmaceutical compositions comprising these compounds.

### DESCRIPTION

Metachromatic leukodystrophy (MLD) (from the greek word *leukos* for "white", *dys* for "lack of", and *troph* for "growth") is an autosomal recessive lysosomal disorder caused by the deficiency in the enzymatic activity of arylsulfatase A (ASA), resulting in impaired degradation of 3-O-sulfogalactosyl ceramide (sulfatide), an essential sphingolipid of myelin. ASA hydrolyzes 3-O-sulfogalactosyl ceramide to galactosylceramide and sulfate and therefore is important for sulfatide degradation. Impairment of ASA function results in increased accumulation of sulfatide which clinically manifests in progressive demyelination and subsequent neurological symptoms resulting in severe debilitation and eventually death of the affected patient. MLD is a rare disorder with a prevalence ranging from 1:40000 to 1:100000. The deficiency in the ASA enzyme is caused by mutations in the ARSA gene in homo- or heterozygosity encoding ASA. Many mutations in the ARSA gene have been identified to date, but not all of these mutations cause the deleterious MLD disease and are likely to be polymorphisms.

Since MLD is caused by defective ASA, most therapeutic approaches have tried to correct the biochemical defect by providing normal ASA. The different methods and sources of wild-type ASA constitute distinct therapeutic approaches. Enzyme replacement therapy (ERT) and HSCT rely on providing normal ASA to the deficient cells, while gene therapy approaches are based on the overexpression of wild-type ASA in different cell types. However, all therapeutic approaches cope with the problem of transporting the active therapeutic across the blood brain barrier (BBB). The BBB controls the migration of components from circulating blood to brain tissue, and, thus, the arterial capillaries in the CNS form a permeability barrier distinct from any other tissue, which harbors endothelial fenestrations between cells. In other words, BBB constitutes the major obstacle to novel therapeutic agents that require reaching the affected neural tissue to treat the neurological manifestations of MLD.

In ERT, the BBB remains a major obstacle for treatment success. Lysosomal ASA is a high molecular weight therapeutic agent, and consequently penetrates the BBB in low amounts lowering its therapeutic effect. Also producing the enzyme in high purity and in large scale recombinantly is complex and costly.

In a gene therapy approach autologous hematopoietic stem cells of a patient are genetically modified to overexpress ARSA using retroviral gene transfer shuttles. The approach was successful in a mouse model of MLD (Biffi A, et al.; J Clin Invest. 2004 Apr; 113(8): 1118-29.) and data of patients treated in a presymptomatic stage of disease are promising. A major limitation of the approach is the risk of increased mutagenesis of cancer related genes due to the retroviral genetic modification of the HSC. Other cell based therapies for replacing ASA try to use microencapsulated recombinant cells, oligodendrocyte progenitor cells, and neural progenitor cells as well as embryonic stem cells. However, also for these approaches safety and tolerability have not been shown in humans so far.

Small molecule-based therapy can potentially overcome limitations of the current therapies for MLD and may also tackle, at multiple levels, different pathogenic and molecular mechanisms involved in MLD. Some small molecules can easily cross the BBB, which is a major hindrance in the ERT strategy. Warfarin, a classical anti-coagulant that functions by competitively inhibiting the multi-unit vitamin K epoxide reductase complex, was shown in mice to reduce the levels of sulfatides by 33%, along with cerebrosides and sphingomyelin in the brain (Sundaram KS, Lev M; J Lipid Res. 1988 Nov; 29(11):1475-9). However, no beneficial effect of the compound could be shown in human patients so far. Therefore, there is an urgent need to develop new small molecule therapeutics for the treatment of MLD that overcome the problems of the prior art therapies mentioned above.

US 2012/0108526 discloses antimicrobial compounds derived from *Epicoccum nigrum* for use in the protection of plants against fungal and bacterial pathogens. Epipyrones A-C were isolated and suggested to have anti-microbial activity and therefore could be used to tackle infection in plants and animals.

WO 2011/071396 discloses antifungal compounds also derived from *Epicoccum nigrum.* The compounds disclosed have antifungal activity.

WO 2005/047275 discloses compounds isolated from *Aspergillus flavipes* during fermentation and their application as anti-bacterial and anti-fungal medicaments.

The above problem is solved by a compound for use in the treatment of a disease associated with a pathological accumulation of sulfatide (3-O-sulfogalactosyl ceramide), wherein the compound is a galactosylceramide-sulfotransferase inhibitor; or a salt, derivative, tautomer, stereoisomer, hydrate, solvate or sugar analogue thereof.

The invention pertains to the inventive concept of tackling ASA deficiency, and the sulfatide accumulation resulting therefrom, by targeting not ASA directly - as many prior art therapeutic approaches did - but by impairing sulfatide synthesis. The inventors therefore developed strategies for the inhibition of the major enzyme responsible for the generation of sulfatide, which is galactosylceramide-sulfotransferase . The inventive concept therefore lies in the inhibition of galactosylceramide-sulfotransferase for treating subjects suffering from a disease caused by the accumulation of sulfatides - preferably MLD, or similar lysosomal storage disorders.

In context of the present invention the term "disease associated with a pathological accumulation of sulfatides" shall pertain to any disease caused by the enzymatic activity of galactosylceramide-sulfotransferase. Particular preferred is that the pathology of the diseases treatable by the invention involves the accumulation of sulfatide - the major product of the enzymatic reaction catalyzed by galactosylceramide-sulfotransferase.

In preferred embodiments of the invention, the disease is a disease of the central nervous system (CNS) and/or peripheral nervous system (PNS). More preferably the disease is a demyelinating disorder. In other preferred embodiments the disease is a leukodystrophy. A leukodystrophy in context with the present invention is preferably selected from metachromatic leukodystrophy, Krabbe disease, adrenoleukodystrophy, Pelizaeus-Merzbacher disease, Canavan disease, Childhood Ataxia with Central Hypomyelination or CACH (also known as Vanishing White Matter Disease), Alexander disease, Refsum disease, and cerebrotendinous xanthomatosis. In most preferred embodiments of the invention the disease is metachromatic leukodystrophy (MLD).

A "sulfotransferase inhibitor" is preferably a compound impairing the enzymatic activity of a polypeptide which catalyzes the transfer of a sulfate group from one compound to the hydroxyl group of another. In preferred embodiments of the present invention the polypeptide which catalyzes the transfer of a sulfate group from one compound to the hydroxyl group of another is a sulfotransferase, or is preferably selected from UDP-galactose:ceramide galactosyltransferase (CGT) and cerebroside sulfotransferase (CST), and most preferably is galactosylceramide-sulfotransferase. A galactosylceramide-sulfotransferase is preferably a human galactosylceramide-sulfotransferase. Human galactosylceramide-sulfotransferase is encoded by the gene *GAL3ST1* (*galactose-3-O-sulfotransferase 1*; HG ID: HGNC:24240, Ensembl gene ID: ENSG00000128242, according to Ensembl release 87 - Dec 2016).

As used herein, the term "sulfotransferase-inhibitor" means a substance that affects a decrease in the amount or rate of sulfotransferase expression or activity. Such a substance can act directly, for example, by binding to sulfotransferase and decreasing the amount or rate of sulfotransferase expression or activity. A sulfotransferase-inhibitor can also decrease the amount or rate of sulfotransferase expression or activity, for example, by binding to sulfotransferase in such a way as to reduce or prevent interaction of sulfotransferase with a substrate or cofactor; by binding to sulfotransferase and modifying it, such as by removal or addition of a moiety; and by binding to sulfotransferase and reducing its stability. A sulfotransferase-inhibitor can also act indirectly, for example, by binding to a regulatory molecule or gene region so as to modulate regulatory protein or gene region function and affect a de-crease in the amount or rate of sulfotransferase expression or activity. Thus, a sulfotransferase-inhibitor can act by any mechanisms that result in decrease in the amount or rate of sulfotransferase expression or activity.

A sulfotransferase-inhibitor can be, for example, a naturally or non-naturally occurring macromolecule, such as a polypeptide, peptide, peptidomimetic, nucleic acid, carbohydrate or lipid. A sulfotransferase-inhibitor further can be an antibody, or antigen-binding fragment thereof, such as a monoclonal antibody, humanized antibody, chimeric antibody, minibody, bifunctional anti-body, single chain antibody (scFv), variable region fragment (Fv or Fd), Fab or F(ab)2. A sulfotransferase-inhibitor can also be polyclonal antibodies specific for sulfotransferase. A sulfotransferase-inhibitor further can be a partially or completely synthetic derivative, analog or mimetic of a naturally occurring macromolecule, or a small organic or inorganic molecule. A preferred sulfotransferase inhibitor is a small molecular compound.

Preferably in some embodiments the sulfotransferase inhibitor is a galactosylceramidesulfotransferase inhibitor.

The sulfotransferase inhibitor of the invention is in preferred embodiments a compound selected from an epipyrone or an epicoccolide; or a salt, derivative, tautomer, stereoisomer, hydrate, solvate or sugar analogue thereof. Epipyrones and epicoccolides of the invention are preferably isolated from, or derivatized from a structure isolated from the fungus *Epicoccum nigrum.*

In particular preferred in context of the present invention is an epipyrone with the following formula:

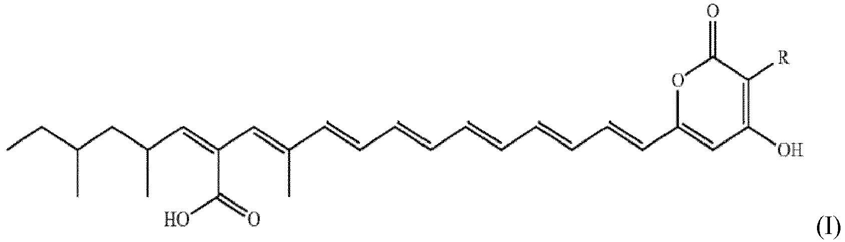

Wherein R is selected from C-β-D-galactopyranose; C-α-D-galactofuranose; C-β-D-galactofuranose; C-β-L-galactopyranose; C-α-L-galactofuranose; C-β-L-galactofuranose; or related pyranose; and a salt, derivative, tautomer, stereoisomer, hydrate, solvate or sugar analogue thereof; or preferably the following epipyrones having the formula 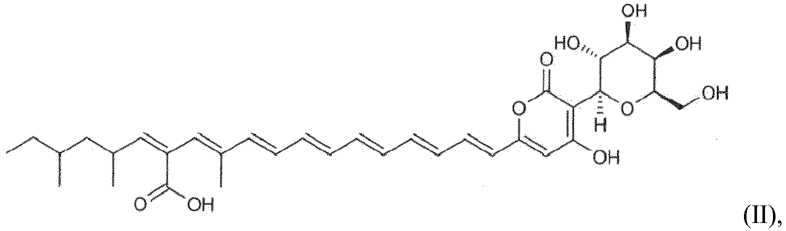 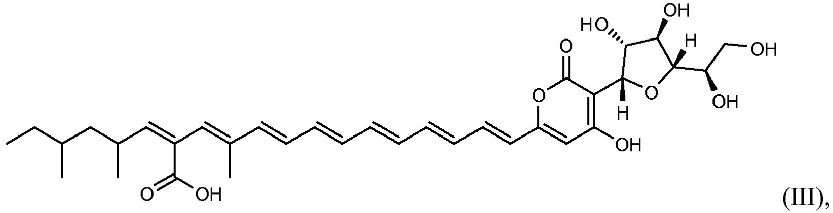 or 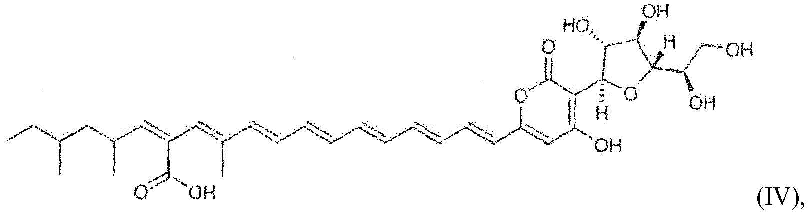 and a salt, derivative, tautomer, stereoisomer, hydrate, solvate or sugar analogue thereof.

The epicoccolide of the invention is preferably a compound having the formula V: 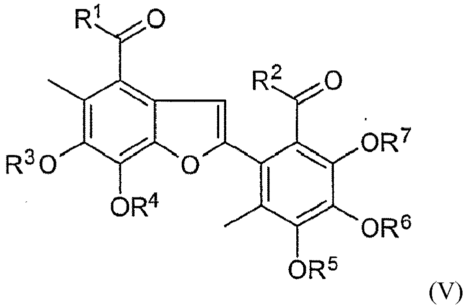 wherein R¹ and R² are, independently of each other, H, OH or -O-(C₁-C₆)- alkyl, and R³, R⁴, R⁵, R⁶ and R⁷ are, independently of each other, H, -(C₁- C₆)-alkyl or -C(O)-(C₁-C₆)-alkyl, or a salt, derivative, tautomer, stereoisomer, hydrate, solvate or sugar analogue thereof.

(C₁-C₆)-Alkyl denotes a straight-chain or branched hydrocarbon group having 1, 2, 3, 4, 5 or 6 carbon atoms, for example methyl (Me), ethyl, n-propyl, isopropyl, tert-butyl or n-hexyl, preferably methyl.
In preferred embodiments the compound of formula (V) comprises H as R¹ and R². Preference is furthermore given to R³, R⁴, R⁵, R⁶ and R⁷ in the compound of the formula (V) being, independently of each other, H or methyl. Particular preference is given to R¹ and R² in the compound of the formula (V) being H and to R³, R⁴, R⁵, R⁶ and R⁷ being, independently of each other, H or methyl.

In preferred embodiments the epicoccolide of the invention is epicoccolide B, which is the compound having the formula (VI) 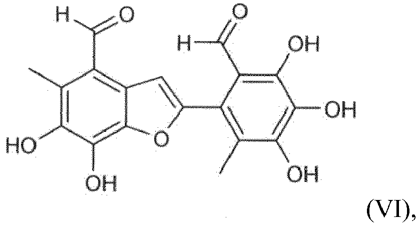 or a salt, derivative, tautomer, stereoisomer, hydrate, solvate or sugar analogue thereof.

The compounds of the invention for use in therapeutic treatments are administered to a patient suffering from a disorder as mentioned herein, in therapeutically effective doses. As used herein, the term "therapeutically effective dose" intends that dose of sulfotransferase inhibitor that achieves a therapeutic effect, and is typically in the range of about 0.05 mg/kg to about 1.0mg/kg/d for both children and adults, and more preferably of about 0.075mg/kg/day to about 0.3 mg/kg/day. The therapeutic dose of compound of the invention can be administered as a single dose or divided doses given in certain intervals of time, for example as two, three, four or more daily doses.

In certain embodiments, the sulfotransferase inhibitors for use in the methods of the present invention further comprise a targeting moiety. Targeting moieties include a protein or a peptide, or other molecule, which directs localization to a certain part of the body, for example and preferably, to the brain or compartments therein. In certain embodiments, sulfotransferase inhibitors for use in the methods of the present invention are attached or fused to a brain targeting moiety. The brain targeting moieties are attached covalently (e.g., direct, translational fusion, or by chemical linkage either directly or through a spacer molecule, which can be optionally cleavable) or non-covalently attached (e.g., through reversible interactions such as avidin:biotin, protein A:IgG, etc.). In other embodiments, the sulfotransferase inhibitors for use in the methods of the present invention thereof are attached to one more brain targeting moieties. In additional embodiments, the brain targeting moiety is attached to a plurality of compounds for use in the methods of the present invention.

A brain targeting moiety associated with a compound enhances brain delivery of such a compound. A number of polypeptides have been described which, when fused to a therapeutic agent, delivers the therapeutic agent through the blood brain barrier (BBB). Non-limiting examples include the single domain antibody FC5 (Abulrob et al. (2005) J. Neurochem. 95, 1201-1214); mAB 83-14, a monoclonal antibody to the human insulin receptor (Pardridge et al. (1995) Pharmacol. Res. 12, 807-816); the B2, B6 and B8 peptides binding to the human transferrin receptor (hTfR) (Xia et al. (2000) J. Virol. 74, 11359-11366); the OX26 monoclonal antibody to the transferrin receptor (Pardridge et al. (1991) J. Pharmacol. Exp. Ther. 259, 66-70); diptheria toxin conjugates. (see, for e.g., Gaillard et al., International Congress Series 1277:185-198 (2005); and SEQ ID NOs: 1-18 of U.S. Pat. No. 6,306,365. The contents of the above references are incorporated herein by reference in their entirety.

Enhanced brain delivery of a compound is determined by a number of means well established in the art. For example, administering to an animal a radioactively labelled compound linked to a brain targeting moiety; determining brain localization; and comparing localization with an equivalent radioactively labelled compound that is not associated with a brain targeting moiety. Other means of determining enhanced targeting are described in the above references.

In another aspect there is also provided a pharmaceutical composition comprising the compound of the invention, the sulfotransferase inhibitor, together with a pharmaceutically acceptable carrier and/or excipient. In some preferred embodiments the pharmaceutical composition is for use in a treatment of diseases as described herein before. Other embodiments of the invention provide the above pharmaceutical compositions comprising two or more compounds according to the invention. In preferred embodiments, the pharmaceutical composition comprises the one or more sulfotransferase inhibitor of the invention in a therapeutically effective dose.

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical), intracerebroventricular, intraparenchymal and transmucosal administration.

The term "intrathecal," as used herein, means introduced into or occurring in the space under the arachnoid membrane which covers the brain and spinal cord. The term "intracerebroventricular" refers to administration of a composition into the ventricular system of the brain, e.g., via injection, infusion, or implantation (for example, into a ventricle of the brain). As used herein, the term "intraparenchymal" can refer to an administration directly to brain tissue. In other instances, intraparenchymal administration may be directed to any brain region where delivery of one or more compounds of the invention is effective to mitigate or prevent one or more of disorders as described herein. Forms of administration directly to brain tissue is on some embodiments preferred.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; anti-bacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a sulfotransferase inhibitor) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of ad-ministration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

In another aspect, there is provided a method for treating a subject suffering from a disorder characterized by the accumulation of sulfatide as described herein elsewhere, the method comprising administering to the subject a therapeutically effective amount of a sulfotransferase inhibitor as defined herein. The sulfotransferase inhibitor is preferably administered to the subject in the form of a pharmaceutical composition as described herein elsewhere.

In another aspect there is provided a method for inhibiting the enzymatic activity of a sulfotransferase, comprising contacting the sulfotransferase with a sulfotransferase-inhibitor as described herein before. In preferred embodiments, the method is for inhibiting the enzymatic activity of a sulfotransferase within a biological cell, preferably a cell of the central nervous system in a mammal. In this embodiment, the method comprises contacting the cell with the sulfotransferase inhibitor of the invention, where "contacting" shall include "introducing into" the cell. Contacting the cell with the sulfotransferase inhibitor may comprise a step of administering to the subject via any of the aforementioned routes a sulfotransferase-inhibitor as described herein.

The method may be performed *in vivo* or *in vitro.*

A subject in context of the present invention is preferably a mammal, more preferably a human. The subject of the invention suffers from a disorder treatable by the compounds of the invention as described herein.

The present invention will now be further described in the following examples with reference to the accompanying figures and sequences, nevertheless, without being limited thereto. For the purposes of the present invention, all references as cited herein are incorporated by reference in their entireties. In the Figures:
- **Figure 1:**: Enzymatic Inhibition of galactosylceramide-sulfotransferase by Epipyrones
- **Figure 2:**: Enzymatic Inhibition of galactosylceramide-sulfotransferase by Epicoccolide B

### EXAMPLES

### Example 1: Galactosylceramide-sulfotransferase -Inhibitory Activity of Epipyrones

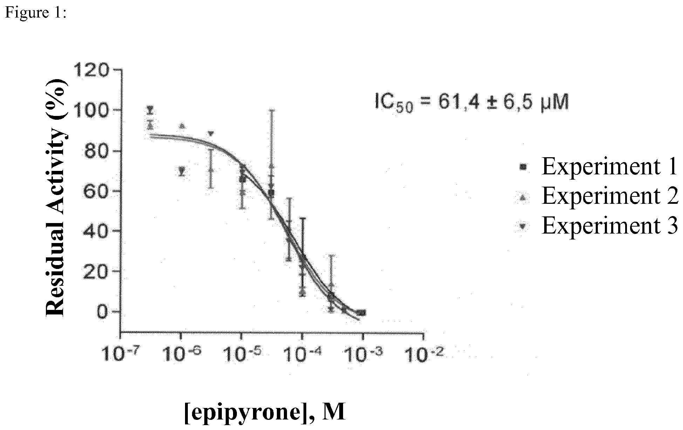

Recombinant purified galactosylceramide-sulfotransferase was incubated 50 µl of 80 mM Tris/Cl pH 7.1, 16 mM MgCl₂, 1,1 µM 3'-phosphoadenosyl-5'-phosphosulfate labelled radioactively with [³⁵S], 1 µg galactosylceramide, 1 % DMSO, 0,1% Triton X-100 for 10 minutes at 37°C. Reaction products were analyzed by thin layer chromatography and the amount of radioactive 3-O-sulfogalactosylceramide was determined as a measure of galactosylceramide-sulfotransferase activity. Results are provided in figure 1. Epipyrones were added in the concentration indicated in figure 1.

### Example 2: Galactosylceramide-sulfotransferase -Inhibitory Activity of Epicoccolide B

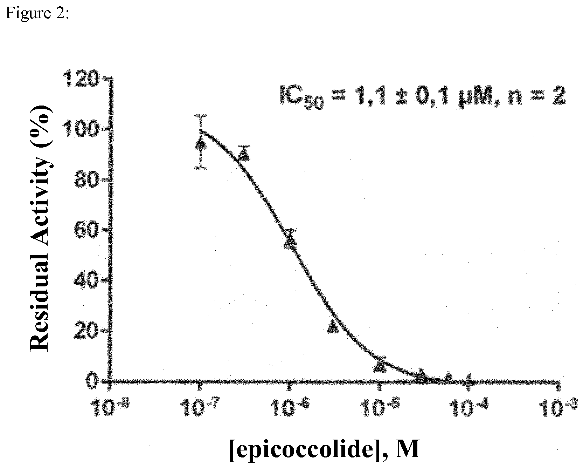

Recombinant purified galactosylceramide-sulfotransferase was incubated 50 µl of 80 mM Tris/Cl pH 7.1, 16 mM MgCl₂, 1,1 µM 3'-phosphoadenosyl-5'-phosphosulfate labelled radioactively with [³⁵S], 1 µg galactosylceramide, 1 % DMSO, 0,1% Triton X-100 for 10 minutes at 37°C. Reaction products were analyzed by thin layer chromatography and the amount of radioactive 3-O-sulfogalactosylceramide was determined as a measure of galactosylceramide-sulfotransferase activity. Results are provided in figure 2. Epicoccolide B was added in the concentration indicated in figure 2.

## Claims

1. A compound for use in the treatment of a disease associated with a pathological accumulation of sulfatides, wherein the compound is a sulfotransferase inhibitor; or a salt, derivative, tautomer, stereoisomer, hydrate, solvate or sugar analogue thereof.

2. The compound for use according to claim 1, wherein the compound is selected from an epipyrone or an epicoccolide; or a salt, derivative, tautomer, stereoisomer, hydrate, solvate or sugar analogue thereof.

3. The compound for use according to claim 2, wherein the epipyrone is selected from a compound having the formula I 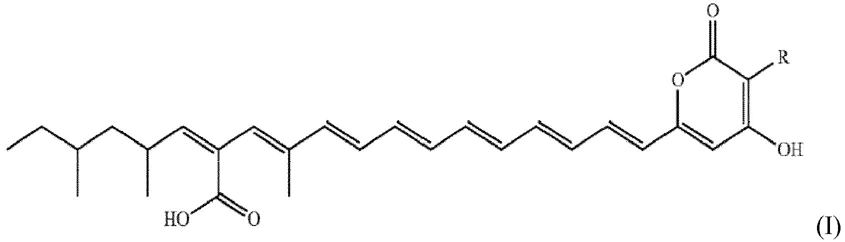 Wherein R is selected from C-β-D-galactopyranose; C-α-D-galactofuranose; C-β-D-galactofuranose; C-β-L-galactopyranose; C-α-L-galactofuranose; C-β-L-galactofuranose; or related pyranose; and a salt, derivative, tautomer, stereoisomer, hydrate, solvate or sugar analogue thereof.

4. The compound for use according to claim 3, which is selected from a compound selected from the group of the compounds having the formula II to IV: 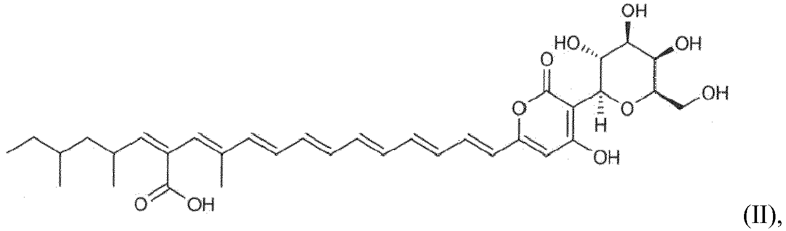 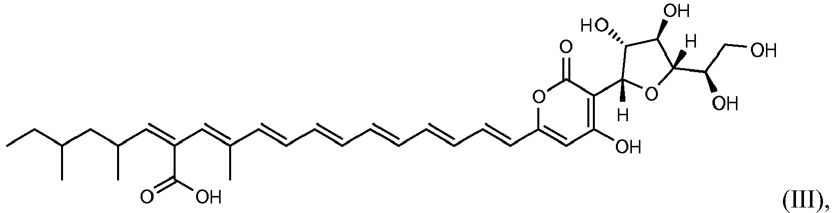 or 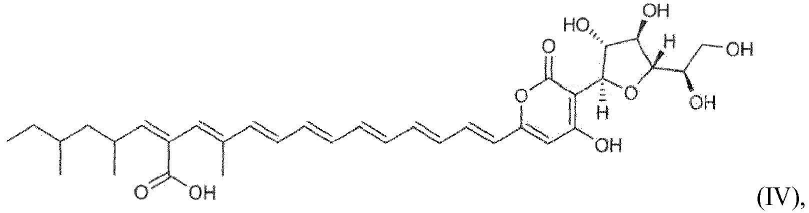 and a salt, derivative, tautomer, stereoisomer, hydrate, solvate or sugar analogue thereof.

5. The compound for use according to claim 2, wherein the epicoccolide is selected from a compound having the formula V, 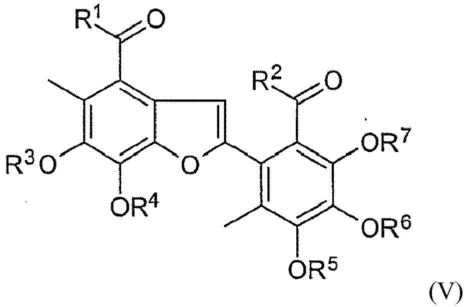 wherein R¹ and R² are, independently of each other, H, OH or -O-(C₁-C₆)- alkyl, and R³, R⁴, R⁵, R⁶ and R⁷ are, independently of each other, H, -(C₁-C₆)-alkyl or -C(O)-(C₁-C₆)-alkyl.

6. The compound for use according to claim 5, wherein the epicoccolide is epicoccolide B, preferably having the formula VI: 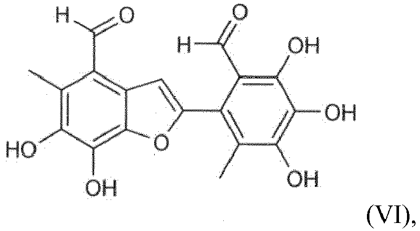 or a salt, derivative, tautomer, stereoisomer, hydrate, solvate or sugar analogue thereof.

7. The compound for use according to any one of claims 1 to 6, wherein the sulfatide is produced by a sulfotransferase.

8. The compound for use according to claim 7, wherein the sulfotransferase is a galactosylceramide-sulfotransferase.

9. The compound for use according to any one of claims 1 to 8, wherein the disease is a disease of the central nervous system.

10. The compound for use according to any one of claims 1 to 9, wherein the disease is a leukodystrophy.

11. The compound for use according to claim 10, wherein the leukodystrophy is a member selected from metachromatic leukodystrophy, Krabbe disease, adrenoleukodystrophy, Pelizaeus-Merzbacher disease, Canavan disease, Childhood Ataxia with Central Hypomyelination or CACH, Alexander disease, Refsum disease, and cerebrotendinous xanthomatosis.

12. The compound for use according to any one of claims 1 to 11, wherein the disease is metachromatic leukodystrophy.

13. A pharmaceutical composition for use according to any one of claims 1 to 12, comprising the compound and a pharmaceutically acceptable carrier and/or excipient.

14. The pharmaceutical composition for use according to claim 13, comprising two or more compounds according to any one of claims 1 to 12.
